Europäisches Patentamt

European Patent Office

Office européen des brevets

(19)

(11) Publication number: **0 385 920**
**A2**

# EUROPEAN PATENT APPLICATION

(21) Application number: 90630055.3

(22) Date of filing: 28.02.90

(51) Int. Cl.⁵: **A61M 25/00**

(30) Priority: 03.03.89 US 319521

(43) Date of publication of application:
**05.09.90 Bulletin 90/36**

(84) Designated Contracting States:
**AT BE CH DE DK ES FR GB GR IT LI LU NL SE**

(71) Applicant: **Fogarty, Thomas J.**
**3270 Alpine Road**
**Portola Valley, California 94028(US)**

(72) Inventor: **Fogarty, Thomas J.**
**3270 Alpine Road**
**Portola Valley, California 94028(US)**
Inventor: **Hermann, George D.**
**11835 Skyline Boulevard**
**Los Gatos, California 95030(US)**
Inventor: **Chin, Albert K.**
**3885 Nelson Drive**
**Palo Alto, California 94306(US)**

(74) Representative: **Schmitz, Jean-Marie et al**
**OFFICE DENNEMEYER S.à.r.l. P.O. Box 1502**
**L-1015 Luxembourg(LU)**

(54) **Variable diameter sheath apparatus for use in body passages.**

(57) A tubular sheath for insertion into a body passage through a reduced diameter access port and expansion to a diameter exceeding that of the access port after introduction into the passage. The sheath is fabricated of an elastomeric material and, when stretched, reduces to an outside diameter significantly less than that of the inside diameter of the body passage. An elongated stylet is extended through the sheath and engaged with the distal portion adjacent the open end thereof to stretch the sheath as it is inserted into a body passage. The elastomeric construction enables an oversized object received within the expanded end portion to be drawn through portions of the sheath of a nominal diameter less than the object.

FIG.1.

EP 0 385 920 A2

## VARIABLE DIAMETER SHEATH APPARATUS FOR USE IN BODY PASSAGES

### Field of the Invention

The present invention relates to a method and apparatus for placing a sheath within a body passage and, more particularly, is concerned with such a method and apparatus for percutaneously inserting a sheath into a blood vessel. In its more specific aspects, the invention is concerned with such a method and apparatus wherein the sheath is percutaneously inserted into a blood vessel in a reduced diameter state and, once within the vessel, expanded to the full interior diameter of the vessel.

### Discussion of the Related Art

In vascular applications, sheaths provide a temporary pathway to the interior of an artery or vein. This pathway facilitates the placement and removal of various instruments by protecting the adjacent tissue, vessel and puncture site. These sheaths often contain a hemostasis valve to prevent leakage of blood out of the proximal end of the sheath. A sideport is often included as well, to provide fluid access for such tasks as infusion of anticoagulants and contrast media.

The most common sheath design for vascular use presently in use is a simple thin-walled tube made of a non-distensible plastic material (e.g. Teflon). Such sheaths are of a fixed diameter and in the larger sizes are relatively inflexible. There have also been efforts to provide such non-distensible sheaths which are highly flexible. U.S. Patent 4,493,711 shows such a construction wherein the sheath is a very thin membrane which is originally inverted within the end of a catheter and, upon reaching the desired situs, everted from the catheter.

The prior art also teaches the use of expandable balloon catheters for removing occlusive objects from body passages. Such catheters distend the body passage with a double-walled balloon and, in use, the occlusive object is drawn into and captured by the balloon. U.S. patents 4,243,040 and 4,469,100 disclose such catheters.

The prior art also teaches the concept of distending the balloon of a dilatation catheter in order to reduce its cross-section for placement within a blood vessel. U.S. Patent 4,315,512 by Thomas J. Fogarty, one of the co-inventors herein, discloses such a catheter. In the case of that catheter, however, the distended balloon does not provide a pathway sheath to facilitate the placement of other instruments, or the removal of occlusions.

### Summary of the Invention

The principal component of the sheath comprises an elongate elastomeric tubular body having a proximal portion of reduced diameter as compared to the body passage within which it is to be received and a distal portion of an expanded diameter substantially equal to that of the passage. The distal portion has an open distal end and contraction means is incorporated into the tubular body to laterally contract the distal portion responsive to movement of the distal portion away from the proximal portion. Restraining means is provided to selectively hold the proximal portion against movement with the distal portion, and motion-imparting means extends longitudinally of the tubular body to selectively move the distal portion away from the proximal portion, thus contracting the distal portion.

The invention provides a method of lining a body passage, such as a blood vessel, with a thin-walled single thickness interior sheath. In the method, an elongate elastomeric sheath is provided having a proximal portion of a nominal outside diameter less than that of the body passage, and a distal portion of an outside diameter substantially equal to that of the passage. The distal portion is formed with an open distal end. In practice of the method, the tube is distended to reduce the outside diameter of the distal portion to approximately that of the proximal portion. The tube is then threaded into the body passage while in the distended condition. Once at the desired location within the passage, the tube is relaxed from distension to permit the distal portion to expand to form a sheath in contact with the interior of the passage.

A principal object of the invention is to provide a flexible variable diameter sheath which may be inserted into a body passage in a reduced-diameter condition and, once in place, expanded to the full size of the passage to seal thereagainst.

Another object of the invention is to provide such a sheath which is of thin-walled single thickness construction.

Still another object of the invention is to provide such a sheath which may be percutaneously inserted into a blood vessel in the reduced-diameter state and, once within the vessel, expanded to several times its reduced-diameter state.

Yet another object of the invention is to provide such a sheath which is fabricated of an elastomeric material with an extended proximal portion of a reduced diameter and a distal portion of an expanded diameter, wherein the distal portion may be reduced to the diameter of the proximal portion by distension.

2

Yet another object related to the latter object, is to provide such a sheath wherein, when in a passive state, the proximal portion assumes the reduced-diameter condition and the distal portion assumes the expanded-diameter condition.

Still another object related to the latter object is to provide such a sheath wherein the proximal portion is expandable to accommodate the passage of an oversized object therethrough.

Another and more general object is to provide such a sheath which is highly flexible so that it may conform to the shape of tortuous body passages through which it is threaded.

Still another and more general object of the invention is to provide such a sheath which may be percutaneously inserted in a reduced-diameter state to minimize the size of the percutaneous puncture and the force exerted thereon by insertion of the sheath.

These and other objects will become more apparent when viewed in conjunction with the following detailed description and accompanying drawings.

Brief Description of the Drawings

Fig. 1 is a perspective view of the sheath, with its distal end partially contracted and its proximal end secured to a hemostasis hub;

Fig. 2 is a side elevational view of the sheath of Fig. 1, with its distal end portion fully distended;

Fig. 3 is a side elevational view of the sheath similar to Fig. 2, with the distal portion of the sheath partially distended;

Fig. 4 is a side elevational view of the sheath similar to Figs. 2 and 3, with the distal end portion of the sheath fully relaxed and expanded;

Fig. 5 is a cross-sectional elevational view of a section of the proximal portion of the sheath, with an instrument in the process of being pushed therethrough, depicting how the proximal portion may passively expand to accommodate a relatively large instrument;

Fig. 6 is a cross-sectional elevational view similar to Fig. 5, with the instrument in the process of being pulled from the sheath, depicting how the proximal portion may passively contract as a relatively large instrument is pulled therefrom;

Fig. 7 is a cross-sectional elevational view of a section of a body passage, illustrating as steps A, B and C, the process of directing the sheath into the passage to capture and remove an occlusive object;

Fig. 8 is a cross-sectional elevational view of a section of a blood vessel, illustrating as steps A through G, the process of percutaneously inserting the sheath into the vessel and using the sheath for the removal of an occlusion;

Fig. 9 is an enlarged cross-sectional view showing a preferred embodiment of the sheath with the distal portion in expanded condition, illustrating the intersecting filaments which extend obliquely to the longitudinal axis of the sheath to facilitate its distension;

Fig. 10 is a cross-sectional elevational view of the distal end of the sheath, illustrating the manner in which the distension stylet of the preferred embodiment is attached to the sheath;

Fig. 11 is a cross-sectional elevational view of the distal end of the sheath, illustrating the manner in which a first alternative embodiment of a distension stylet may be attached to the sheath;

Fig. 12 is a cross-sectional elevational view of the distal end of the sheath, illustrating the manner in which a second alternative embodiment of a distension stylet may be attached to the sheath; and

Fig. 13 is an elevational view, with parts thereof broken away, illustrating the full length of the sheath with the preferred embodiment distension stylet incorporated thereinto and extending through the hemostasis hub secured to the proximal end of the sheath.

Description of the Preferred Embodiments

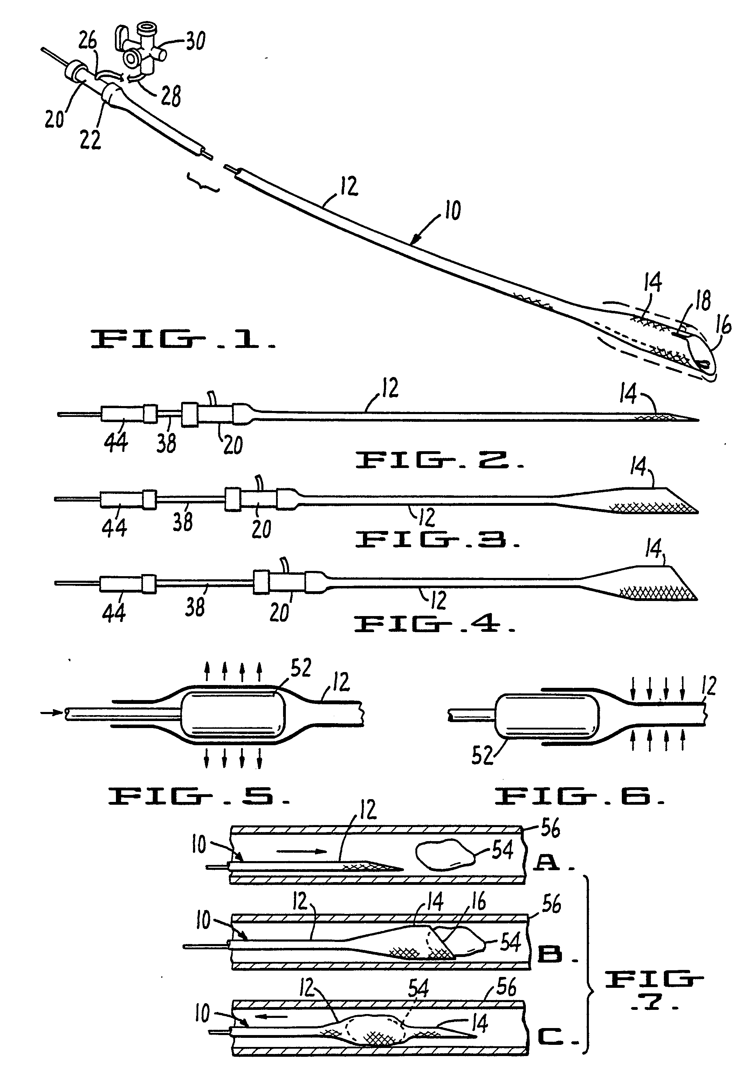

Referring now to Fig. 1, the sheath is designated therein in its entirety by the numeral 10. The sheath is elongate and comprises a reduced-diameter proximal portion 12 and an expanded-diameter distal portion 14. The distal portion is proportioned to expand to an outside diameter (see the phantom line illustrations in Fig. 1) aproximately equal to the inside diameter of the vessel within which the catheter is to be used. The proximal portion has an outside diameter approximately one third that of the distal portion. The distal portion has an open distal end 16 which extends obliquely to the longitudinal axis of the catheter. A longitudinally-extending slit 18 is formed in the distal end at the edge thereof most close to the proximal portion 12.

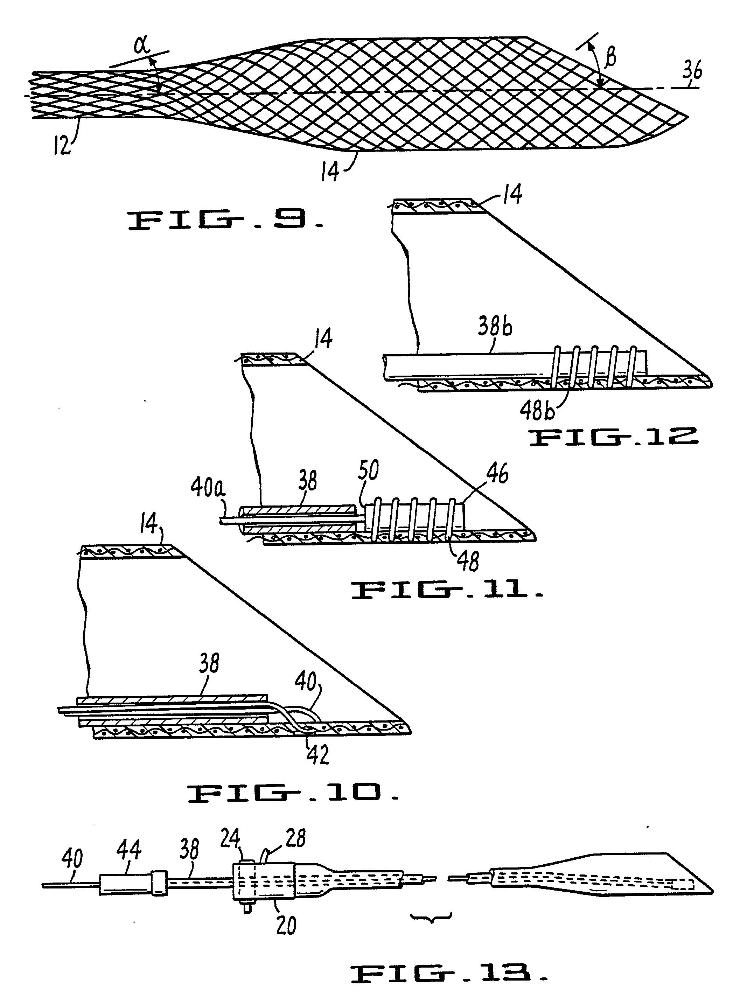

A hemostasis hub 20 is received within the proximal end 22 of the portion 12. This hub is of conventional construction and provided to prevent the leakage of blood out of the proximal end of the device. It includes a valve 24 (Fig. 13) to permit the passage of elongate elements into the catheter, and a sideport 26 to provide fluid access to the catheter for tasks such as the infusion of anticoagulants and contrast media. As shown, a sideport tube 28 is secured in sealed fluid communication with the sideport 26 and a stopcock value 30 is secured to the tube 28. The valve is of conventional construction and provides means whereby the tube **28** may be selectively opened or closed.

The internal construction of the preferred embodiment of the sheath may best be seen from Fig. 9. As there illustrated, the wall of the sheath comprises a tubular braid **32** encapsulated within a coating **34** of high-elongation silicon polymer. The braid is a woven structure of flexible generally inelastic polyester monofilaments **35**, such as Dacron. While the proportions of the sheath may vary, depending upon the size and type of body passage within which it is intended to be used, the following is a typical example for arterial use:

Proximal portion **12**: .1000 inches ID (in relaxed condition)

Distal Portion **14**: .2500 to .3000 inches ID (in relaxed condition)

Wall thickness: .0200 inches

Braid **32**: 6 mil. polyester monofilament manufactured by Atkins Pearce Manufacturing of Covington, Kentucky under No. GP-4819425.

Coating **34**: High-elongation silicone elastomer manufactured by Dow Corning of Midland, Michigan under No. Q7-2213.

Length of distal portion **14** 2 to 3 inches.

In this example, the filaments **35** within the proximal portion **12** extend at an angle $\alpha$ of approximately 10 to 20 degrees relative to the longitudinal axis, designated **36**, of the sheath and those within the distal portion **14** extend at an angle $\beta$ of approximately 35 to 50 degrees relative to said axis. These angles and the foregoing dimensions are for the sheath when in the relaxed condition, with the distal end fully expanded. In the contracted condition, the distal portion **14** is reduced to a diameter approximately equal to that of the proximal portion and the angle $\beta$ is reduced to that of the angle $\alpha$.

The filaments **35** and their angular relationship relative to the longitudinal axis **36** of the sheath both reinforce the elastomeric coating **34** and facilitate contraction and expansion of the sheath. During contraction of the distal portion **14** the angle $\beta$ decreases. During expansion of the proximal portion **12**, the angle $\alpha$ increases. The filaments also serve to control expansion, retraction and elongation of the sheath.

The distal end **16** extends at an oblique angle relative to the axis **36**. This angle is chosen so as to increase the area of the open distal end, as compared to what would occur if the angle were 90 degrees. In the preferred embodiment, the angle is chosen so as to be approximately equal to that of the angle $\beta$ so that the distal-most filaments run generally parallel to the distal edge.

Elongation of the sheath to contract the distal portion **14** is provided by extending a stylet through the sheath to a secure compression-imparting connection adjacent the distal end **16**. Fig. 10 shows a preferred embodiment where in the stylet, designated **38**, is tubular and secured to the distal portion **14** by a double filament **40** secured to the distal portion by loop connection **42** which extends around the braided filament within the portion **14**. Both the stylet **38** and the wire **40** extend through the full length of the sheath **10** and from the proximal portion thereof, as can be seen from Fig. 13. As there shown, it will be seen that the stylet **38** extends slidably through the hemostasis hub **20** and the valve **24** therein and that a collet grip **44** is clampingly secured to the proximal end of the stylet **38**. The wire **40** extends fully through the stylet **38** and from the proximal end thereof. It should be understood that the collet grip **44** does not grip the wire **40** and that, accordingly, the stylet **38** is freely longitudinally moveable on the wire.

In use, the hemostasis hub **20** serves as a grip to the restrain the proximal portion **12** against movement as the distal portion **14** is longitudinally extended by the stylet **38** for lateral contraction. As viewed in Fig. 13, this process would be carried out by manually holding the hub **38** as the grip **44** is moved to the right to extend the stylet into compression-imparting relationship to the distal end of the portion **14**. Conversely, the distal portion is expanded by simply relaxing compressive force on the grip **44** to permit the stylet **38** to move to the left as the distal portion **14** expands by virtue of its elastomeric character. The tubular stylet embodiment of Fig. 10 also has the advantage that the stylet may be completely removed from the sheath to increase its flexibility. Such removal is achieved simply by pulling the grip **44** fully to the left, as viewed in Fig. 13. Such removal of the stylet leaves the wire **40** in place so that the stylet may later be threaded back into the sheath and into compression-imparting relationship to the distal end thereof.

The first alternative embodiment of Fig. 11 corresponds to that of Fig. 10, with the exception of the construction of the wire within the stylet and the manner in which it is secured to the distal end of the sheath. Accordingly, the elements of this embodiment corresponding to those of the Fig. 10 embodiment are designated by like numerals. The securing wire, designated **40a**, for this embodiment is of a monofilament construction and formed with an enlarged end **46** which is secured to the body of the portion **14** by a helical tie **48**. The tie **48** extends around the filaments of the tubular braid **32** to provide a secure connection. The end **46** provides an abutment shoulder **50** against which the distal end of the stylet **38** may seat to extend the portion **14** for lateral contraction. Other than these differences in construction, the operation of the Fig. 11 embodiment corresponds to that of Fig.

10.

The second alternative embodiment of Fig. 12 differs from the embodiment of Fig. 11 in that the stylet 38b is solid, rather than tubular, and not removable from the sheath. In Fig. 12, the stylet is secured to the distal portion of the sheath by helical loop ties 48b. The operation of the Fig. 12 embodiment differs from that of the embodiments of Figs. 10 and 11 in that the stylet is not removable from the sheath and that, accordingly, flexibility of the sheath cannot be increased by removing the stylet.

Figs. 2, 3 and 4 illustrate the Fig. 10 embodiment of the sheath and its operation when moving from the fully-contracted condition to the fully-expanded condition. As shown in Fig. 2, the proximal end of the sheath is restrained by the hub 20 while the stylet 38 is held extended through the grip 44 to distend the distal portion 14 to a contracted cross-section approximating that of the proximal portion 12. In Fig. 3, the compression imparted to the distal portion 14 by the stylet 38 is relaxed to permit the distal portion to partially expand. Fig. 4 shows the compression imparted by the stylet fully relaxed to permit the distal portion 14 to fully expand.

Figs. 5 and 6 depict the sheath as an instrument 52 is being moved therethrough and illustrate how the proximal portion 52 may passively expand and contract responsive to movement of the instrument. In Fig. 5, the instrument is being moved to the right and the proximal portion 12 is expanding, as depicted by the lateral arrow lines. In Fig. 6 the instrument is being removed and the proximal portion 12 is contracting, as depicted by the lateral arrow lines. These figures demonstrate that the passive contraction of the sheath allows it to contract in regions that the user does not want dilated (e.g. arterial puncture, adjacent muscle, skin).

Fig. 7 shows the sheath in the process of removing an occlusive object 54 from a body passage 56. As shown in Step A, the sheath has been actively contracted by extending the stylet and is in the process of moving through the passage and toward the object. Step B shows the sheath with the distal portion in the expanded condition and the object being drawn thereinto. In Step C, the object has been drawn into the sheath and the proximal portion of the sheath has passively expanded to accommodate the object. The distal portion of the sheath in Step C has been actively contracted by extending the stylet 38.

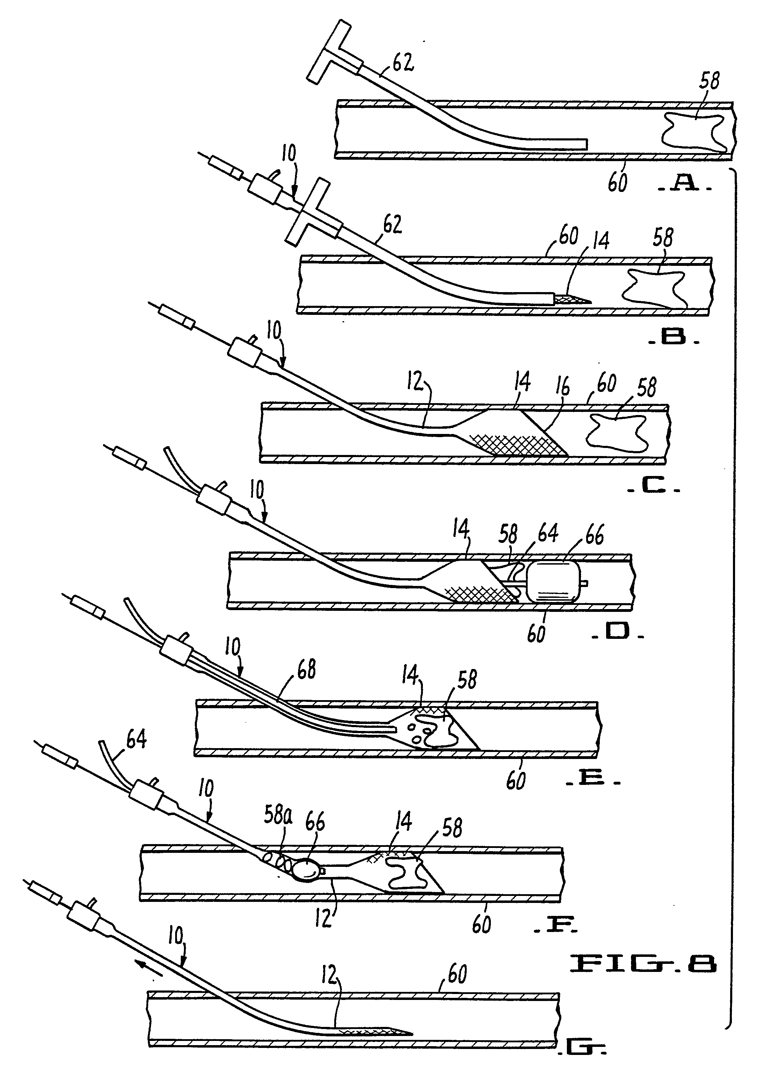

Fig. 8 shows a typical sequence of operation for percutaneously using the sheath to remove an occlusive plaque or thrombosis 58 from a blood vessel 60. Step A shows a standard introducer (peelaway) sheath 62 which has been percutaneously inserted into the vessel in preparation of the process. In Step B, the sheath 10 has been advanced into the vessel through the sheath 62, with the distal portion 14 in the distended contracted condition. Step C shows the introducer (peelaway) sheath withdrawn and the distal portion 14 relaxed to an expanded condition lining the interior of the vessel 60. In Step D, an inflatable embolectomy catheter 64 has been extended through the sheath 10, out of the distal portion 14, and past the thrombosis 58. It should be appreciated that the embolectomy catheter is so extended with the balloon thereof, designated 66 in a contracted condition. As shown in Step D, the balloon has been expanded by inflation and is shown in the process of drawing the thrombosis 58 into the distal portion 14 of the sheath. Step E shows the sheath 10 after the embolectomy catheter 64 has been withdrawn therefrom and a plaque disruption tool 68 has been inserted into the sheath to fragment the thrombosis 58 for removal. The tool may take any suitable form, such as a high-speed mechanical cutter or a laser. Step F shows the sheath with the disruption tool removed therefrom and an embolectomy catheter 64 in the process of pushing fragmented pieces, designated 58a, of the thrombosis through the sheath. As shown in Step F, the balloon 66 of the embolectomy catheter 64 is inflated so as to engage and slightly expand the walls of the proximal portion 12. It should be appreciated that Steps E and F would be repeated until the thrombosis 58 was fully removed from the vessel 60. Step G shows the sheath 10 in the process of being removed from the vessel 60 after the process of Fig. 8 has been completed. As shown in Step G, the distal portion 14 has been distended to contract it to the reduced-diameter condition.

Conclusion

The invention is not limited to the specific embodiments and uses described. It may find use in literally any application where it is desired to provide an enlarged sheath within a body passage, with a minimum of distention to the passage during insertion of the sheath, or the drawing of enlarged objects therethrough. It may also be used wherever it is desired to shield the body passage from a process being carried out therein, such as the fragmentation of an occlusive object.

Claims

1. A variable diameter sheath for insertion into a body passage, said sheath comprising:

(a) a elongate flexible tubular body having a proximal portion of a reduced diameter as compared to the diameter of the body passage and a distal portion of an expanded diameter as compared to said proximal portion, said distal portion having an open distal end;

(b) contraction means incorporated into the tubular body to laterally contract the distal portion responsive to the movement of the distal portion away from the proximal portion;

(c) restraining means to selectively hold the proximal portion against movement with the distal portion; and,

(d) motion imparting means extending longitudinally of the tubular body to selectively move the distal portion away from the proximal portion.

2. A sheath according to Claim 1 wherein the motion imparting means comprises:

(a) an elongate generally incompressible element extending along the tubular body for longitudinal movement relative thereto: and,

(b) connecting means adapted to secure said element in compression imparting relationship to the distal portion adjacent the distal end thereof.

3. A sheath according to Claim 2 wherein the elongate element extends over the full length of the tubular body and from the proximal portion, said sheath further comprising grip means on the elongate element to the outside of the proximal portion for manual gripping to impart compressive force to the element.

4. A sheath according to Claim 2 wherein:

(a) the elongate element comprises a tubular stylet extending longitudinally through the tubular body; and,

(b) the connecting means comprises a wire extending slidably through the stylet and secured to the distal portion adjacent the open distal end thereof.

5. A sheath according to Claim 1 wherein the tubular body is elastomeric and the contraction means comprises a plurality of intersecting flexible generally inelastic filaments incorporated into the distal portion, said filaments extending obliquely to the longitudinal axis of the body.

6. A sheath according to Claim 5 wherein the open distal end extends obliquely to the longitudinal axis of the tubular body.

7. A sheath according to Claim 6 wherein the distal end is longitudinally slit at the edge thereof most close to the proximal portion.

8. A sheath according to Claim 1 wherein the tubular body is elastomeric and the proximal portion is expandible to accommodate oversized objects drawn thereinto from the distal portion.

9. A variable diameter sheath for insertion into a body passage, said sheath comprising:

(a) a elongate flexible tubular body having a proximal portion of a reduced diameter as compared to the diameter of the body passage and a distal portion of an expanded diameter as compared to said proximal portion, said distal portion having an open distal end;

(b) contraction means incorporated into the tubular body to laterally contract the distal portion responsive to the movement of the distal portion relative to the proximal portion;

(c) restraining means to selectively hold the proximal portion against movement with the distal portion; and,

(d) motion imparting means extending longitudinally of the tubular body to selectively contract the distal portion by moving the distal portion relative to the proximal portion.

10. A sheath according to Claim 9 wherein the tubular body is elastomeric and the proximal portion is expandible to accommodate oversized objects drawn thereinto from the distal portion.

11. A elongate variable diameter sheath having a proximal portion of a reduced nominal diameter and an open ended distal portion contiguous with said proximal portion of a diameter of from two to three times that of the proximal portion, said sheath comprising:

(a) a continuous braided tubular body extending over the length of said proximal and distal portions, said body being defined by intersecting flexible generally inelastic filaments extending obliquely to the longitudinal axis of the sheath; and,

(b) an impermeable elastomeric coating on said tubular body.

12. A sheath according to Claim 11 wherein the filaments within the distal portion are expanded relative to those within the proximal portion.

13. A sheath according to Claim 12 further comprising contraction means incorporated into the sheath to contract the distal portion to approximately the diameter of the proximal portion responsive to movement of the distal portion longitudinally relative to the proximal portion.

14. A sheath according to Claim 13 wherein the contraction means comprises:

(a) restraining means to selectively hold the proximal portion against movement with the distal portion;

(b) an elongate generally incompressible element extending along the sheath for longitudinal movement relative thereto: and,

(c) connecting means adapted to secure said element in compression imparting relationship to the distal portion of the sheath.

15. A sheath according to Claim 14 wherein the elongate element extends over the full length of the sheath and has a section extending past the proximal portion thereof, further comprising grip means on said section for manual gripping to impart compressive force to the element.

16. A sheath according to Claim 15 wherein:

(a) the elongate element comprises a tubular stylet extending longitudinally through the sheath; and,

(b) the connecting means comprises a wire secured to the distal end portion and extending slidably through the stylet.

17. A variable diameter sheath for insertion into a body passage, said sheath comprising:

(a) an elongate flexible tubular body of an outside diameter over at least a portion of its length substantially equal to that of said passage, said body having an open distal end;

(b) contraction means incorporated into the tubular body to laterally contract said portion while in the body passage to an outside diameter substantially less than that of said passage responsive to extending said portion axially to a stretched condition; and,

(c) motion imparting means to selectively extend said portion to the stretched condition and release said portion from said condition while in the body passage.

18. A sheath according to Claim 17 wherein the motion imparting means comprises:

(a) an elongate generally incompressible element extending along the tubular body for longitudinal movement relative thereto; and,

(b) connecting means adapted to secure said element in compression imparting relationship to the body adjacent the open distal end thereof.

19. A sheath according to Claim 18 wherein the elongate element extends over the full length of the tubular body, said sheath further comprising grip means on the elongate element to the outside of the tubular body for manual gripping to impart compressive force to the element.

20. A sheath according to Claim 18 wherein:

(a) the elongate element comprises a tubular stylet extending longitudinally through the tubular body; and,

(b) the connecting means comprises a wire extending slidably through the stylet and secured to the body adjacent the open distal end thereof.

21. A sheath according to Claim 17 wherein the tubular body is elastomeric and the contraction means comprises a plurality of intersecting flexible generally inelastic filaments incorporated into the distal portion, said filaments extending obliquely to the longitudinal axis of the body.

22. A elongate variable diameter sheath comprising:

(a) a continuous braided tubular body defined by intersecting flexible generally inelastic filaments extending obliquely to the longitudinal axis of the sheath, said body having an open distal end; and,

(b) an impermeable elastomeric coating on said tubular body.

23. A sheath according to Claim 22 further comprising control means to selectively stretch the tubular body to reduce the diameter of the sheath.

24. A sheath according to Claim 23 wherein the control means comprises:

(a) an elongate generally incompressible element extending along the body for longitudinal movement relative thereto: and,

(b) connecting means adapted to secure said element in compression imparting relationship to the body adjacent the open distal end thereof.

25. A sheath according to Claim 24 wherein the elongate element extends over the full length of the sheath, further comprising grip means on said element for manual gripping to impart compressive force to the element.

26. A sheath according to Claim 24 wherein:

(a) the elongate element comprises a tubular stylet extending longitudinally through the sheath; and,

(b) the connecting means comprises a wire extending slidably through the stylet and secured to the body adjacent the open distal end thereof.

FIG.1.

FIG.2.

FIG.3.

FIG.4.

FIG.5.

FIG.6.

FIG.7.

62

58

10

60   .A.

62

60  14   58

10

.B.

12   14   16   60   58

10

.C.

14   58   64   66

10

60   .D.

10   68   14   58

64

60   .E.

10   58a  66   14   58

12   60   .F.

10

12   60

FIG.8

.G.

FIG. 9.

FIG. 10.

FIG. 11.

FIG. 12

FIG. 13.